# EUROPÄISCHE PATENTSCHRIFT

(11) **EP 2 921 156 B1**
(45) Veröffentlichungstag und Bekanntmachung des Hinweises auf die Patenterteilung: **02.10.2019**
(21) Anmeldenummer: 14160841.4
(22) Anmeldetag: 20.03.2014
(51) Int. Cl.: A61K 6/083

(54) **Monomermischung zur Herstellung von Dentalwerkstoffen**
Monomer mixture for the preparation of dental materials
Mélange de monomères pour la fabrication de matériaux dentaires

(43) Veröffentlichungstag der Anmeldung: 23.09.2015
(73) Patentinhaber: Ivoclar Vivadent AG, 9494 Schaan (LI)
(72) Erfinder: Moszner, Norbert, 9495 Triesen (LI); Burtscher, Peter, 6830 Rankweil (AT); Gianasmidis, Alexandros, 9435 Heerbrugg (CH)
(74) Vertreter: Uexküll & Stolberg

(56) Entgegenhaltungen:
- EP-A1- 2 233 123
- EP-A1- 2 649 981
- US-A- 3 084 436
- US-A1- 2010 068 679

## Beschreibung

Die vorliegende Erfindung betrifft eine Monomermischung zur Herstellung Dentalwerkstoffen, die sich insbesondere zur Verwendung als dentales Füllungsmaterial, dentaler Befestigungszement oder dentales Beschichtungsmaterial eignen.

Dentale Komposite enthalten gewöhnlich eine polymerisierbare organische Matrix und einen oder mehrere Füllstoffe. Als polymerisierbare organische Matrix wird in den meisten Fällen eine Mischung von Monomeren, Initiator-Komponenten, Stabilisatoren und Pigmenten verwendet, wobei als Monomere oft Mischungen von Dimethacrylaten eingesetzt werden. Die Aushärtung derartiger Materialien kann durch thermische, redoxinitiierte oder lichtinduzierte radikalische Polymerisation erfolgen. In zunehmendem Maße werden auch acide Monomere zur Herstellung von Dentalmaterialien verwendet. Diese verleihen den Materialien selbstätzende Eigenschaften und verbessern ihre Haftung an der natürlichen Zahnsubstanz.

Häufig verwendete Monomere sind Dimethacrylate wie 2,2-Bis[4-(2-hydroxy-3-methacryloyloxypropyl)phenyl]propan (Bis-GMA) und 1,6-Bis-[2-methacryloyloxyethoxycarbonylamino]-2,4,4-trime-thylhexan (UDMA), Bismethacryloyloxymethyltricyclo[5.2.1.]-decan (TCDMA), Decandiol-1,10-dimethacrylat (D₃MA) und Triethylenglycoldimethacrylat (TEGDMA). Bis-GMA ist das bekannteste aromatische Dimethacrylat, das nach seinem Entwickler auch als Bowen-Monomer oder Bowen-Harz bezeichnet wird. Bis-GMA hat eine relativ hohe Viskosität und lässt sich daher nur schwer mit Füllstoffen mischen. Es wird daher praktisch immer mit niedrigviskosen Monomeren kombiniert, wobei sich eine Basismischung von Bis-GMA mit TEGDMA als Standard für kommerzielle Produkte etabliert hat.

Obwohl Bis-GMA und TEGDMA nicht systemisch toxisch sind, da ihre LD₅₀-Werte über 5.000 µg/kg Körpergewicht (Ratte) liegen, zeigen sie doch einige unerwünschte Effekte. So haben Bis-GMA und TEGDMA eine vergleichsweise hohe Cytotoxizität, und es ist bekannt, dass TEGDMA zu Genmutationen in DNA-Molekülen von Säugetieren führt (vgl. G. Schmalz, D Arenholt-Bindslev, Biocompatibility of Dental Materials,Springer-Verlag, Berlin Heidelberg 2009, S. 110 ff).

Der Erfindung liegt die Aufgabe zugrunde, Monomermischungen zur Herstellung von Dentalwerkstoffen bereitzustellen, die eine geringe Cytotoxität aufweisen und die in der Lage sind, TEGDMA, Bis-GMA und deren Mischungen in Dentalwerkstoffen zu ersetzen. Die Monomermischungen sollen sich durch gute Härtungseigenschaften, geringe Viskosität, geringe Wasserlöslichkeit und eine hohe Reaktivität auszeichnen und die homogene Einarbeitung auch hoher Füllstoffmengen ermöglichen. Die daraus hergestellten Polymere sollen in ihren mechanischen Eigenschaften mit denen von Polymeren auf der Basis von TEGDMA und Bis-GMA übereinstimmen.

Diese Aufgabe wird erfindungsgemäß durch eine Monomermischung gelöst, die als schwerflüchtiges Monomethacrylat 2 bis 50 Gew.-% p-Cumylphenoxyethylenglycolmethacrylat (CMP-1E), als hochviskoses Dimethacrylat 5 bis 65 Gew.-% TMX-UDMA (ein Additionsprodukt von HEMA und Hydroxypropylmethacrylat (HPMA) mit α ,α,α',α'-Tetramethyl-m-xylylendiisocyanat (TMXDI)) und/oder 1,6-Bis-[2-methacryloyloxyethoxycarbonylamino]-2,4,4-trimethylhexan (UDMA), und als niedrigviskoses Dimethacrylat 5 bis 55 Gew.-% Bismethacryloyloxymethyltricyclo[5.2.1.]decan (TCDMA), Glycerindimethacrylat (GDMA) und/oder Decandiol-1,10-dimethacrylat (D3 MA) enthält und die kein TEGDMA und kein Bis-GMA enthält.

Erfindungsgemäß werden unter schwerflüchtigen Monomeren Verbindungen mit einem Siedepunkt > 150°C bei Normaldruck verstanden. Der Siedepunkt kann z.B. mit einer Destillationsapparatur bestimmt werden. Unter hochviskosen Monomeren werden Stoffe mit einer Viskosität ≥ 5 Pa·s, vorzugsweise 5 bis 10.000 Pa·s und besonders bevorzugt 5 bis 2.000 Pa·s und unter niedrigviskosen Monomeren Stoffe mit einer Viskosität ≤ 300 mPa·s, vorzugsweise 1 bis 300 mPa·s und besonders bevorzugt 30 bis 300 mPa·s verstanden, wobei die Viskosität mit einem Kapillar- (niedrigviskos) oder Rotationsviskosimeter (hochviskos) bei einer Temperatur von 25°C bestimmt wird.

Die erfindungsgemäße, radikalisch polymerisierbare Monomermischung enthält:
2 bis 50 Gew.-%, bevorzugt 5 bis 45 Gew.-% und besonders bevorzugt 10 bis 30 Gew.-% des schwerflüchtigen Monomethacrylats p-Cumylphenoxyethylenglycolmethacrylat (CMP-1E);
5 bis 65 Gew.-%, bevorzugt 8 bis 60 Gew.-% und besonders bevorzugt 25 bis 50 Gew.-% mindestens eines hochviskosen polyfunktionellen Methacrylats, das aus TMX-UDMA (ein Additionsprodukt von HEMA und Hydroxypropylmethacrylat (HPMA) mit α ,α,α',α'-Tetramethyl-m-xylylendiisocyanat (TMXDI)) und 1,6-Bis-[2-methacryloyloxyethoxycarbonylamino]-2,4,4-trimethylhexan (UDMA) ausgewählt ist,; und
5 bis 55 Gew.-%, bevorzugt 10 bis 50 Gew.-% und besonders bevorzugt 20 bis 45 Gew.-% eines niedrigviskosen polyfunktionellen Methacrylats, das aus Bismethacryloyloxymethyltricyclo[5.2.1.]decan (TCDMA), Glycerindimethacrylat (GDMA) und/oder Decandiol-1,10-dimethacrylat (D₃MA) ausgewählt ist.

Die obigen Gewichtsangaben beziehen sich auf die Gesamtmasse der Monomermischung.

Besonders bewährt hat sich eine Monomermischung, die 20 Gew.-% CMP-1E, 20 Gew.-% GDMA, 20 Gew.-% TMX-UDMA, 25 Gew.-% UDMA und 15 Gew.-% D₃MA enthält.

Die erfindungsgemäße Monomermischung eignet sich besonders zur Herstellung von Dentalwerkstoffen, insbesondere von Füllungskompositen, Befestigungszementen und dentalen Beschichtungsmaterialien, wie z.B. Fissurenversieglern. Die erfindungsgemäßen Monomermischungen und Dentalwerkstoffe enthalten kein TEGDMA und kein Bis-GMA. Im folgenden werden unter Dentalwerkstoffen Zusammensetzungen verstanden, die neben der oben definierten Monomermischung mindestens eine weitere Komponente enthalten, vorzugsweise mindestens einen Initiator für die radikalische Polymerisation, besonders bevorzugt einen Photoinitiator.

Es wurde überraschenderweise gefunden, dass die erfindungsgemäßen Monomermischungen TEGDMA, Bis-GMA und deren Mischungen in Dentalmaterialien ersetzten können, ohne das Härtungsverhalten, die mechanischen Eigenschaften oder die Gebrauchsmaterialien der Werkstoffe zu beeinträchtigen. Die erfindungsgemäße Monomermischung zeichnet sich gegenüber TEGDMA und Bis-GMA durch eine geringere Cytotoxizität aus.

Zur Herstellung von Dentalwerkstoffen kann die erfindungsgemäße Monomermischung mit weiteren radikalisch polymerisierbaren Monomeren versetzt werden.

Als zusätzliche radikalisch polymerisierbare Monomere oder Mischungen radikalisch polymerisierbarer Monomere sind Methacrylate bevorzugt, besonders bevorzugt Mischungen aus mono- und polyfunktionellen Methacrylaten, ganz besonders bevorzugt aus mono- und difunktionellen Methacrylaten.

Bevorzugte mono- oder polyfunktionelle Methacrylate sind Methyl-, Ethyl-, 2-Hydroxyethyl-, Butyl-, Benzyl-, Tetrahydrofurfuryl- oder Isobornyl(meth)acrylat, ethoxy- oder propoxyliertes Bisphenol-A-Dimethacrylat, wie z.B. das Bisphenol-A-Dimethacrylat SR-348c mit 3 Ethoxygruppen oder 2,2-Bis[4-(2-methacryloxypropoxy)phenyl]propan, Di-, Tri- oder Tetraethylenglycoldimethacrylat, Trimethylolpropantrimethacrylat, Pentaerythrittetramethacrylat, sowie Glycerindi- und trimethacrylat, 1,4-Butandioldimethacrylat, oder 1,12-Dodecandioldimethacrylat.

Alternativ oder zusätzlich kann die erfindungsgemäße Monomermischung neben den oben genannten Monomeren ein oder mehrere säuregruppenhaltige radikalisch polymerisierbare Monomere (Haftmonomere) als Zusatzmonomere enthalten. Diese verleihen den Werkstoffen selbsthaftende und/oder selbstätzende Eigenschaften.

Bevorzugte säuregruppenhaltige Monomere sind polymerisationsfähige Carbonsäuren, Phosphonsäuren, Phosphorsäureester, und Sulfonsäuren.

Bevorzugte Carbonsäuren sind Maleinsäure, Acrylsäure, Methacrylsäure, 2-(Hydroxymethyl)acrylsäure, 4-(Meth)acryloyloxyethyltrimellitsäure, 10-Methacryloyloxydecylmalonsäure, N-(2-Hydroxy-3-methacryloyloxypropyl)-N-phenylglycin bzw. 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure oder 2-[4-(Dihydroxyphosphoryl)-2-oxa-butyl]-acrylsäureethyl- und -2,4,6-trimethylphenylester.

Bevorzugte acide polymerisationsfähige Phosphorsäureester sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)-propylsulfonsäure.

Besonders bevorzugte Haftmonomere sind 4-(Meth)acryloyloxyethyltrimellitsäure, 2-[4-(Dihydroxyphosphoryl)-2-oxa-butyl]-acrylsäureethyl- oder -2,4,6-trimethylphenylester und 10-Methacryloyloxydecyl-dihydrogenphosphat.

Gemäß einer Ausführungsform der Erfindung enthalten die Dentalwerkstoffe neben der erfindungsgemäßen Monomermischung keine weiteren Monomere.

Die erfindungsgemäßen Monomermischungen enthalten vorzugsweise auch einen Initiator für die radikalische Polymerisation. Zur Härtung der Materialien werden z.B. im Falle von indirekten Füllungsmaterialien thermische Initiatoren, wie z.B. Dibenzoylperoxid (DBPO), oder Derivate der Barbitursäure eingesetzt, wie z.B. Trimethylbarbitursäure. Für bei Härtung bei Raumtemperatur werden die Peroxide mit Aminen wie N,N-Dimethyl-sym.-xylidin oder N,N-Dimethyl-p-toluidin und die Barbitursäurederivate mit Perverbindungen wie z.B. Kaliumperoxosulfat oder Perestern kombiniert.

Als Photoinitiatoren für lichthärtende Materialien sind α-Diketone bevorzugt, wie z.B. Campherchinon (1,7,7-Trimethylbicyclo[2.2.1]heptan-2,3-dion) (CQ) und 9,10-Phenanthrenchinon. Photoinitiatoren werden in der Regel zusammen mit Aminen wie 4-(N,N-Dimethylamino)-benzoesäureethylester als Reduktionsmittel eingesetzt. Dualhärtenden Dentalmaterialien enthalten eine Mischung von einem Photoinitiator und einem Redox-Initiatorsystem.

Erfindungsgemäß sind Dentalwerkstoffe bevorzugt, die als Initiator für die radikalische Polymerisation eine Kombination aus mindestens einem Thioharnstoffderivat und mindestens einer Bisacyldialkylgermanium-Verbindung enthalten. Dieses Initiatorsystem erlaubt die Herstellung von Werkstoffen, die lagerstabil sind, insbesondere auch in Gegenwart acider Komponenten, die nach der Härtung verbesserte mechanische Eigenschaften aufweisen und farbstabil sind. Dual härtende Dentalwerkstoffe enthalten als zusätzliche Initiatorkomponente weiterhin ein Peroxid, vorzugsweise ein Hydroperoxid.

Erfindungsgemäß bevorzugte Thioharnstoffderivate werden in US 3,991,008 (Spalte 2, Zeile 35 bis Spalte 3, Zeile 14) und in EP 1 754 465 A1 (Absatz [0009]) beschrieben. Besonders bevorzugte Thioharnstoffderivate sind Methyl-, Ethyl-, Allyl-, Butyl-, Hexyl-, Octyl-, Benzyl-, 1,1,3-Trimethyl-, 1,1-Diallyl, 1,3-Diallyl-, 1-(2-Pyridyl)-2-thioharnstoff, Acetyl-, Propanoyl-, Butanoyl-, Pentanoyl-, Hexanoyl-, Heptanoyl-, Octanoyl, Nonanoyl, Decanoyl und Benzoyl-thioharnstoff, wobei Acetyl- und Hexanoylthioharnstoff ganz besonders bevorzugt sind.

Bevorzugte Bisacyldialkylgermanium-Verbindungen werden in EP 1 905 413 A1, EP 1 905 415 A1 und EP 2 103 297 A1 beschrieben, wobei Bisacylgermane gemäß der Formel (II) der EP 1 905 413 A1 besonders bevorzugt sind. Ganz besonders bevorzugte Bisacyldialkygermanium-Verbindungen sind Bisbenzoyldiethylgermanium, Bisbenzoyldimethylgermanium, Bisbenzoyldibutylgermanium, Bis(4-methoxybenzoyl)dimethylgermanium und Bis(4-methoxybenzoyl)diethylgermanium, wobei Bis(4-methoxybenzoyl)diethylgermanium am meisten bevorzugt ist.

Bevorzugte Hydroperoxide sind 1,1,3,3,-Tetramethylbutylhydroperoxid, t-Butylperoxid, Cumolhydroperoxid, Pinanhydroperoxid, p-Menthanhydroperoxid, Diisopropylbenzolhydroperoxid und t-Amylhydroperoxid, wobei Cumolhydroperoxid besonders bevorzugt ist.

Photopolymerisierbare Dentalwerkstoffe liegen vorzugsweise als einkomponentige Systeme vor, d.h. in Form einer Mischung, die sämtliche Bestandteile des Dentalwerkstoffs enthält. Sie enthalten als Initiator ausschließlich einen Photoinitiator und lassen such durch Bestrahlung mit Licht härten.

Dualhärtende Dentalwerkstoffe enthalten neben dem Photoinitiator zusätzlich ein Peroxid, vorzugsweise Hydroperoxid als Oxidationsmittel. Dualhärtende Werkstoffe liegen vorzugsweise in Form von zwei getrennten Komponenten vor, da sonst eine vorzeitige Aushärtung erfolgen würde, wobei die erste Komponente das (Hydro)peroxid und die zweite Komponente das Thioharnstoffderivat enthält. Das Thioharnstoffderivat dient als Reduktionsmittel (Beschleuniger). Die Komponenten werden dementsprechend auch als Katalysator- und als Beschleunigerpaste bezeichnet.

Die Härtung der dual härtenden Werkstoffe kann durch das Mischen von Katalysator- und Beschleunigerpaste ausgelöst werden. Die Zusammensetzung wird so eingestellt, dass sie nach dem Mischen der Pasten noch für einige Minuten (sog. Verarbeitungszeit) verarbeitungsfähig bleibt, nach der Verarbeitung aber schnell aushärtet. Die Verarbeitungs- und Härtungszeit lassen sich vor allem durch Art und Konzentration an (Hydro)peroxid, Thioharnstoff-Derivat und ggf. durch die Zugabe weiteren Komponenten wie Übergangsmetallredoxkatalysator und Inhibitor einstellen.

In der Regel verläuft eine durch Redox-Initiatorsysteme ausgelöste Polymerisation langsamer als eine Photopolymerisation. Dementsprechend lassen sich bei dualhärtenden Werkstoffen Überschüsse leicht entfernen, indem die durch Bestrahlung ausgelöste Photopolymerisation erst nach der Überschussentfernung erfolgt.

Weiterhin enthalten die erfindungsgemäßen Dentalwerkstoffe vorzugsweise auch organische oder besonders bevorzugt anorganische partikuläre Füllstoffe. Bevorzugt sind Füllstoffe auf der Basis von Oxiden, wie SiO₂, ZrO₂ und TiO₂ bzw. Mischoxiden aus SiO₂, ZrO₂, ZnO und/oder TiO₂, nanopartikuläre oder mikrofeine Füllstoffe, wie pyrogene Kieselsäure oder Fällungskieselsäure (gewichtsmittlere Partikelgröße von 10-1.000 nm) sowie Minifüllstoffe, wie Quarz-, Glaskeramik- oder röntgenopake Glaspulver von z.B. Barium- oder Strontiumaluminiumsilikatgläsern (gewichtsmittlere Partikelgröße von 0,2-10 µm). Weitere bevorzugte Füllstoffe sind röntgenopake Füllstoffe, wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid (gewichtsmittlere Partikelgröße von 10-1.000 nm).

Zur Verbesserung des Verbundes zwischen den Füllstoffpartikeln und der vernetzten Polymerisationsmatrix können SiO₂-basierende Füllstoffe mit methacrylat-funktionalisierten Silanen, wie z.B. 3-Methacryloyloxypropyltrimethoxysilan, oberflächenmodifiziert werden. Zur Oberflächenmodifizierung von nichtsilikatischen Füllstoffen, z.B. von ZrO₂ oder TiO₂, können auch funktionalisierte saure Phosphate, wie z.B. 10-Methacryloyloxydihydrogenphospaphat eingesetzt werden.

Füllstoffhaltige Dentalwerkstoffe eignen sich besonders als dentale Füllungskomposite, Zemente und Beschichtungsmaterialien. Besonders bevorzugt sind Werkstoffe, die nur Füllstoffe mit einer maximalen Partikelgröße von weniger als 600 nm enthalten. Diese eignen sich besonders als dentale Zemente.

Gegebenenfalls können die erfindungsgemäß eingesetzten Zusammensetzungen weitere Additive enthalten, vor allem Stabilisatoren, wie z.B. Polymerisationsstabilisatoren, Farbmittel, mikrobiocide Wirkstoffe, fluoridionenabgebende Additive, optische Aufheller, Fluoreszensmittel, Weichmacher, Übergangsmetallredoxkatalysatoren und/oder UV-Absorber.

Als Übergangsmetallredoxkatalysatoren eignen sich besonders Verbindungen von Übergangsmetallen, die mindestens zwei stabile Wertigkeitsstufen haben. Das sind vor allem Verbindungen der Elemente Kupfer, Eisen, Vanadium, Nickel oder Kobalt, wobei Kupferverbindungen besonders bevorzugt sind und diese ganz besonders bevorzugt als gut organolösliche Verbindungen, wie z.B. als Acetylacetonat, Naphthenat oder 2-Ethyl-hexanoat, eingesetzt werden. Diese Katalysatoren beschleunigen die Redoxreaktion von Oxidations- und Reduktionsmittel und damit die Radikalbildung, d.h. z.B die Redoxreaktion von Hydroperoxid und Thioharnstoffderivat.

Erfindungsgemäß sind solche Dentalwerkstoffe besonders bevorzugt, die die folgende Zusammensetzung haben:
(a) 12,0 bis 75 Gew.-%, besonders bevorzugt 19 bis 56 Gew.-% der erfindungsgemäßen Monomermischung,
(b) 20 bis 85 Gew.-%, besonders bevorzugt 40 bis 80 Gew.-% Füllstoff(e),
(c) 0,05 bis 4 Gew.-%, vorzugsweise 0,1 bis 2,0 Gew.-% Initiator für die radikalische Polymerisation und ggf.
(d) 0,1 bis 5,0 Gew.-% besonders bevorzugt 0,1 bis 2,0 Gew.-% Additiv(e) .

Die obigen Angaben beziehen sich auf die Gesamtmasse des Dentalwerkstoffs.

Die erfindungsgemäßen Dentalwerkstoffe enthalten als Komponente (c) vorzugsweise 0,01 bis 4,0 Gew.-%, besonders bevorzugt 0,1 bis 2,0 Gew.-% Thioharnstoffderivat(e) und 0,001 bis 1,0 Gew.-%, besonders bevorzugt 0,005 bis 0,5 Gew.-% Bisacyldialkylgermanium-Verbindung(en). Dualhärtende Werkstoffe enthalten vorzugsweise zusätzlich 0,1 bis 3,0 Gew.-%, besonders bevorzugt 0,1 bis 2,0 Gew.-% Hydroperoxid(e).

Die erfindungsgemäßen Dentalwerkstoffe können als Komponente (e) 0 bis 15 Gew.-%, besonders bevorzugt 0-10 Gew.-% eines oder mehrerer säuregruppenhaltiger Haftmonomere enthalten.

Die erfindungsgemäßen Dentalwerkstoffe können in ein- oder zweikomponentiger Form bereitgestellt werden. Dualhärtende Werkstoffe sind vorzugsweise zweikomponentig, d.h. sie enthalten zwei getrennte Komponenten, die vor der Anwendung miteinander gemischt werden. Die Zusammensetzung der Komponenten wird so gewählt, dass nach dem Mischen Werkstoffe mit der oben definierten Gesamtzusammensetzung erhalten werden.

Besonders bevorzugt sind solche Dentalwerkstoffe, welche aus den genannten Stoffen bestehen. Weiterhin sind solche Werkstoffe bevorzugt, bei denen die einzelnen Stoffe jeweils aus den oben genannten bevorzugten und besonders bevorzugten Stoffen ausgewählt sind. Ganz besonders bevorzugt sind Werkstoffe, die keine Amine wie z.B. Amin-Beschleuniger, enthalten.

Die erfindungsgemäßen Dentalwerkstoffe eignen sich besonders als dentale Zemente, Füllungskomposite, Beschichtungs- und Verblendmaterialien sowie als Materialien zur Herstellung von Inlays, Onlays, Kronen und Brücken. Die Werkstoffe enthalten vorzugsweise nur Füllstoffe mit einer maximalen Partikelgröße von < 600 nm. Sie gestatten die Herstellung von Dentalwerkstoffen mit geringer Oberflächenrauhigkeit und hohem Glanz sowie ausgezeichneter Abrasionsstabilität.

Die Dentalwerkstoffe eignen sich primär zur intraoralen Anwendung durch den Zahnarzt zur Restauration geschädigter Zähne (klinische Materialien). Sie können aber auch extraoral eingesetzt werden, beispielsweise bei der Herstellung oder Reparatur von Dentalrestaurationen (technische Materialien).

Die Erfindung wird im Folgenden anhand von Ausführungsbeispielen näher erläutert.

### Ausführungsbeispiele

### Beispiele 1-3:

### Lichthärtende Komposite auf der Basis einer erfindungsgemäßen Monomermischung

Entsprechend der nachfolgend aufgeführten Tabelle 1 wurden Komposite (alle Angaben in Masse-%) auf der Basis von 44,5% eines silanisierten SiO₂-Mischoxides mit einem Gehalt von 30 Gew.-% ZrO₂) und 20 % Ytterbiumfluorid hergestellt. In den Beispielen wurde zum einen eine erfindungsgemäße Monomermischung (20% CMP-1E, 20% GDMA, 20% TMX-UDMA, 25% UDMA und 14,5% D₃MA sowie 0,5% BHT als Stabilisator) und zum Vergleich eine Monomermischung auf der Basis von Bis-GMA und TEGDMA verwendet (20% Bis-GMA, 20% TEGDMA, 20% CMP-1E, 25 %UDMA und 14,5% D₃MA sowie 0,5% BHT als Stabilisator). Als Initiatorsystem waren die in Tabelle 1 angegebenen Komponenten enthalten. Die Komposite sind mittels eines Kneters (Firma Linden) hergestellt worden.

Anschließend wurden die mechanischen Eigenschaften der Werkstoffe bestimmt. Die Messung der Biegefestigkeit (BF) und des E-Moduls (EM) erfolgte gemäß der Norm ISO-4049 (Dentistry - Polymerbased filling, restorative and luting materials). Die Messwerte sind in Tabelle 2 angegeben.

Zur Messung der Vickershärte (VH) wurden Metallformen (h = 2mm, Ø = 10mm) mit Komposit gefüllt und mit einer PET-Folie abgedeckt. Die Polymerisation erfolgte durch Bestrahlen von oben mit einer Polymerisationslampe (LED Bluephase; Fa. Ivoclar Vivadent AG:; 10 s bei 650 mW/cm²). Die Prüfkörper wurden nach der Herstellung im Trockenschrank bei 37°C für 24 h gelagert und dann die belichtete Oberseite der Prüfkörper zuerst mit einem 2.500er, danach mit einem 4.000er Schleifpapier plan angeschliffen und schließlich mit Polierpaste poliert. Die Messung der Vickershärte erfolgte an der polymerisierten Oberseite mit einem Universalhärteprüfgerät (Modell ZHU0.2; Fa. Zwick/Röll). Pro Prüfkörper wurden je 3 Einzelmessungen durchgeführt. Die resultierenden Mittelwerte sind in der Tabelle 2 angegeben.

Die Ergebnisse belegen, dass die erfindungsgemäßen Monomermischungen die Herstellung von Dentalwerkstoffen erlauben, die ähnliche Eigenschaften wie Werkstoffe auf der Basis der bewährten Monomere Bis-GMA/TEGDMA aufweisen. Komposite, die einen Ge-Photoinitiator (Ivocerin®) in Kombination mit einem Thioharnstoffderivat enthalten, weisen im Vergleich zu Kompositen, die Campherchinon im Kombination mit einem Amin-Beschleuniger (EMBO) enthalten, eine signifikant erhöhte Vickershärte auf.

**Tabelle 1: Dentalkomposite (Angaben in Gew.-%)**

| **Komponente** | **Bsp. 1** | **Bsp. 2** | **Bsp. 3*)** |
|---|---|---|---|
| Monomermischung | 33,95 | 35,45 | 35,45 |
| SiO₂-Mischoxid¹⁾ | 44,5 | 44,5 | 44,5 |
| YbF₃ | 20 | 20 | 20 |
| Ivocerin®²⁾ | 0,05 | - | - |
| ATU³⁾/Cu⁴⁾ | 1,50 (65 ppm) | - | - |
| CQ⁵⁾ | - | 0,02 | 0,02 |
| EMBO⁶⁾ | - | 0,03 | 0,03 |

| | | | |
|---|---|---|---|
| *) Vergleichsbeispiel 1) SiO₂ mit einem Gehalt von 30 Gew.-% ZrO₂ 2) Bis-(4-methoxybenzoyl)diethylgermanium (Photoinitiator, Ivoclar Vivadent) 3) 1-Acetylthioharnstoff (Beschleuniger) 4) Cu-Acetylacetonat (Cu-Gehalt in ppm) 5) Campherchinon (Photoinitiator) 6) (4-Dimethylamino)benzoesäureethylester (Aminbeschleuniger) 7) Stabilisator | | | |

**Tabelle 2: Mechanische Eigenschaften der Komposite**

| **Bsp.** | **Biegefestigkeit [MPa]** | **E-Modul [MPa]** | **Vickershärte (MPa)** |
|---|---|---|---|
| 1 | 91,0±7,9 | 4870±197 | 242,8±10.1 |
| 2 | 98,7±7,9 | 4910±268 | 207,7±7,7 |
| 3^{*)} | 105±5,5 | 5120+152 | 198,4±10,0 |

| | | | |
|---|---|---|---|
| *) Vergleichsbeispiel | | | |

## Patentansprüche

1. Monomermischung zur Herstellung von Dentalwerkstoffen, die
als schwerflüchtiges Monomethacrylat 2 bis 50 Gew.-% p-Cumyl-phenoxyethylenglycolmethacrylat (CMP-1E),
als hochviskoses Dimethacrylat 5 bis 65 Gew.-% TMX-UDMA (ein Additionsprodukt von HEMA und Hydroxypropylmethacrylat (HPMA) mit α,α,α',α'-Tetramethyl-m-xylylendiisocyanat (TMXDI)) und/oder 1,6-Bis-[2-methacryloyloxyethoxy-carbonylamino]-2,4,4-trimethylhexan (UDMA), und
als niedrigviskoses Dimethacrylat 5 bis 55 Gew.-% Bismethacryloyloxymethyltricyclo[5.2.1.]decan (TCDMA), Glycerindimethacrylat (GDMA) und/oder Decandiol-1,10-dimethacrylat (D₃MA) enthält und
die kein TEGDMA und kein Bis-GMA enthält.

2. Monomermischung nach Anspruch 1, die
5 bis 45 Gew.-% und bevorzugt 10 bis 30 Gew.-% schwerflüchtiges Monomethacrylat;
8 bis 60 Gew.-% und bevorzugt 25 bis 50 Gew.-% hochviskoses Dimethacrylat; und
10 bis 50 Gew.-% und bevorzugt 20 bis 45 Gew.-% niedrigviskoses Dimethacrylat enthält.

3. Monomermischung nach Anspruch 1 oder 2, die 20 Gew.-% CMP-1E, 20 Gew.-% GDMA, 20 Gew.-% TMX-UDMA, 25 Gew.-% UDMA und 15 Gew.-% D₃MA enthält.

4. Monomermischung nach einem der Ansprüche 1 bis 3, die zusätzlich einen Initiator für die radikalische Polymerisation enthält.

5. Monomermischung nach Anspruch 4, die als Initiator für die radikalische Polymerisation eine Kombination von einem Thioharnstoffderivat und einer Bisacyldialkylgermanium-Verbindung enthält.

6. Monomermischung nach Anspruch 5, die als Thioharnstoffderivat Methyl-, Ethyl-, Allyl-, Butyl-, Hexyl-, Octyl-, Benzyl-, 1,1,3-Trimethyl-, 1,1-Diallyl, 1,3-Diallyl-, 1-(2-Pyridyl)-2-thioharnstoff, Acetyl-, Propanoyl-, Butanoyl-, Pentanoyl-, Hexanoyl-, Heptanoyl-, Octanoyl, Nonanoyl, Decanoyl, Benzoyl-thioharnstoff oder eine Mischung davon enthält.

7. Monomermischung nach Anspruch 5 oder 6, die als Bisacyldialkygermanium-Verbindung Bisbenzoyldiethylgermanium, Bisbenzoyldimethylgermanium, Bisbenzoyldibutylgermanium, Bis(4-methoxybenzoyl)dimethylgermanium, Bis(4-methoxybenzoyl)diethylgermanium oder eine Mischung davon enthält.

8. Monomermischung nach einem der Ansprüche 5 bis 7, die zusätzlich ein Peroxid oder vorzugsweise ein Hydroperoxid enthält.

9. Monomermischung nach Anspruch 8, die als Hydroperoxid 1,1,3,3,-Tetramethylbutylhydroperoxid, t-Butylperoxid, Cumolhydroperoxid, Pinanhydroperoxid, p-Menthanhydroperoxid, Diisopropylbenzolhydroperoxid, t-Amylhydroperoxid oder eine Mischung davon enthält.

10. Monomermischung nach einem der Ansprüche 1 bis 9, die zusätzlich organischen oder anorganischen partikulären Füllstoff enthält.

11. Monomermischung nach Anspruch 10, die nur Füllstoff mit einer maximalen Partikelgröße von kleiner 600 nm enthält.

12. Monomermischung nach einem der Ansprüche 1 bis 11, die
(a) 12,0 - 75 Gew.-%, bevorzugt 19 - 56 Gew.-% Monomermischung gemäß einem der Ansprüche 1 bis 3,
(b) 20 - 85 Gew.-%, bevorzugt 40 - 80 Gew.-% Füllstoff(e),
(c) 0,05 - 4 Gew.-%, vorzugsweise 0,1 - 2,0 Gew.-% Initiator für die radikalische Polymerisation und ggf.
(d) 0,1 - 5,0 Gew.-%, bevorzugt 0,1 - 2,0 Gew.-% Additiv(e) enthält.

13. Monomermischung gemäß einem der Ansprüche 1 bis 12 zur intraoralen Anwendung als dentaler Zement, Füllungskomposit, Beschichtungsmaterial oder Verblendmaterial.

14. Verwendung einer Monomermischung gemäß einem der Ansprüche 1 bis 12 zur Herstellung von Inlays, Onlays, Kronen oder Brücken.

## Claims

1. Monomer mixture for the preparation of dental materials which comprises
2 to 50 wt % p-cumylphenoxyethylene glycol methacrylate (CMP-1E) as a low-volatile monomethacrylate,
5 to 65 wt % TMX-UDMA (an addition product of HEMA and hydroxypropyl methacrylate (HPMA) with α,α,α',α'-tetramethyl-*m*-xylylene diisocyanate (TMXDI)) and/or 1,6-bis-[2-methacryloyloxyethoxycarbonylamino]-2,4,4-trimethylhexane (UDMA) as a highly viscous dimethacrylate, and
5 to 55 wt% bismethacryloyloxymethyltricyclo[5.2.1.]decane (TCDMA), glycerol dimethacrylate (GDMA) and/or decanediol-1,10-dimethacrylate (D₃MA) as a low-viscosity dimethacrylate and
which comprises neither TEGDMA nor bis-GMA.

2. Monomer mixture according to claim 1 which comprises
5 to 45 wt % and preferably 10 to 30 wt % of low-volatile monomethacrylate;
8 to 60 wt % and preferably 25 to 50 wt % of highly viscous dimethacrylate; and
10 to 50 wt % and preferably 20 to 45 wt % of low-viscosity dimethacrylate.

3. Monomer mixture according to claim 1 or 2 which comprises 20 wt % CMP-1E, 20 wt % GDMA, 20 wt % TMX-UDMA, 25 wt % UDMA and 15 wt % D₃MA.

4. Monomer mixture according to one of claims 1 to 3 which additionally comprises an initiator for the radical polymerization.

5. Monomer mixture according to claim 4 which comprises a combination of a thiourea derivative and a bisacyldialkylgermanium compound as the initiator.

6. Monomer mixture according to claim 5 which comprises methyl, ethyl, allyl, butyl, hexyl, octyl, benzyl, 1,1,3-trimethyl, 1,1-diallyl, 1,3-diallyl, 1-(2-pyridyl)-2-thiourea, acetyl, propanoyl, butanoyl, pentanoyl, hexanoyl, heptanoyl, octanoyl, nonanoyl, decanoyl, benzoyl thiourea or a mixture thereof as the thiourea derivative.

7. Monomer mixture according to claim 5 or 6 which comprises bisbenzoyldiethylgermanium, bisbenzoyldimethylgermanium, bisbenzoyldibutylgermanium, bis(4-methoxybenzoyl)dimethylgermanium, bis(4-methoxybenzoyl)diethylgermanium or a mixture thereof as the bisacyldialkylgermanium compound.

8. Monomer mixture according to one of claims 5 to 7 which additionally comprises a peroxide or preferably a hydroperoxide.

9. Monomer mixture according to claim 8 which comprises 1,1,3,3-tetramethylbutyl hydroperoxide, *t*-butyl hydroperoxide, cumene hydroperoxide, pinane hydroperoxide, p-menthane hydroperoxide, diisopropylbenzene hydroperoxide, *t*-amyl hydroperoxide or a mixture thereof as the hydroperoxide.

10. Monomer mixture according to one of claims 1 to 9 which additionally comprises an organic or inorganic particulate filler.

11. Monomer mixture according to claim 10 which comprises filler solely having a maximum particle size of less than 600 nm.

12. Monomer mixture according to one of claims 1 to 11 which comprises
(a) 12-75 wt %, preferably 19-56 wt % of the monomer mixture according to one of claims 1 to 3,
(b) 20-85 wt %, preferably 40-80 wt % of filler(s),
(c) 0.05-4 wt %, preferably 0.1-2.0 wt % of initiator for the radical polymerization and optionally
(d) 0.1-5.0 wt %, preferably 0.1-2.0 wt % of additive(s).

13. Monomer mixture according to one of claims 1 to 12 for intraoral application as a dental cement, filling composite, coating material or veneering material.

14. Use of a monomer mixture according to one of claims 1 to 12 for preparing inlays, onlays, crowns or bridges.

## Revendications

1. Mélange de monomères pour préparation de matériaux de dentisterie, lequel mélange contient
- en tant que monométhacrylate peu volatil, de 2 à 50 % en poids de méthacrylate de p-cumyl-phénoxy-éthylèneglycol (CMP-1E),
- en tant que diméthacrylate fortement visqueux, de 5 à 65 % en poids de TMX-UDMA {un produit d'addition de méthacrylate d'hydroxy-éthyle (HEMA) et de méthacrylate d'hydroxy-propyle (HPMA) avec de 1'α,α,α',α'-tétraméthyl-m-xylylène-diisocyanate (TMXDI)} et/ou de 1,6-bis(2-méthacryloyloxy-éthoxy-carbonyl-amino)-2,4,4-triméthyl-hexane (UDMA),
- et en tant que diméthacrylate faiblement visqueux, de 5 à 55 % en poids de bis(méthacryloyloxy-méthyl)-tricyclo[5.2.1]décane (TCDMA), de diméthacrylate de glycérol (GDMA) et/ou de diméthacrylate de décane-1,10-diol (D₃MA),
mais ne contient pas de TEG-DMA, ni de Bis-GMA.

2. Mélange de monomères conforme à la revendication 1, qui contient
- de 5 à 45 % en poids, et de préférence de 10 à 30 % en poids, de monométhacrylate peu volatil,
- de 8 à 60 % en poids, et de préférence de 25 à 50 % en poids, de diméthacrylate fortement visqueux,
- et de 10 à 50 % en poids, et de préférence de 20 à 45 % en poids, de diméthacrylate faiblement visqueux.

3. Mélange de monomères conforme à la revendication 1 ou 2, qui contient 20 % en poids de CMP-1E, 20 % en poids de GDMA, 20 % en poids de TMX-UDMA, 25 % en poids d'UDMA et 15 % en poids de D₃MA.

4. Mélange de monomères conforme à l'une des revendications 1 à 3, qui contient en outre un amorceur de polymérisation par voie radicalaire.

5. Mélange de monomères conforme à la revendication 4, qui contient, en tant qu'amorceur de polymérisation par voie radicalaire, une combinaison d'un dérivé de thiourée et d'un composé de type bis (acyl)-dialkyl-germanium.

6. Mélange de monomères conforme à la revendication 5, qui contient, en tant que dérivé de thiourée, de l'un des suivants : méthyl-thiourée, éthyl-thiourée, allyl-thiourée, butyl-thiourée, hexyl-thiourée, octyl-thiourée, benzyl-thiourée, 1,1,3-triméthyl-thiourée, 1,1-diallyl-thiourée, 1,3-diallyl-thiourée, 1-(pyridin-2-yl)-2-thiourée, acétyl-thiourée, propanoyl-thiourée, butanoyl-thiourée, pentanoyl-thiourée, hexanoyl-thiourée, heptanoyl-thiourée, octanoyl-thiourée, nonanoyl-thiourée, décanoyl-thiourée, et benzoyl-thiourée, ou un mélange de ces dérivés.

7. Mélange de monomères conforme à la revendication 5 ou 6, qui contient, en tant que composé de type bis(acyl)-dialkyl-germanium, du bis(benzoyl)-diéthyl-germanium, du bis(benzoyl)-diméthyl-germanium, du bis(benzoyl)-dibutyl-germanium, du bis(4-méthoxy-benzoyl)-diméthyl-germanium, du bis(4-méthoxy-benzoyl)-diéthyl-germanium, ou un mélange de ces composés.

8. Mélange de monomères conforme à l'une des revendications 5 à 7, qui contient en outre un peroxyde, ou mieux, un hydroperoxyde.

9. Mélange de monomères conforme à la revendication 8, qui contient, en tant qu'hydroperoxyde, du 1,1,3,3-tétraméthyl-butyl-hydroperoxyde, du tertiobutyl-peroxyde, de l'hydroperoxyde de cumène, de l'hydroperoxyde de pinane, de l'hydroperoxyde de para-menthane, de l'hydroperoxyde de diisopropyl-benzène, du tertioamyl-hydroperoxyde, ou un mélange de ces composés.

10. Mélange de monomères conforme à l'une des revendications 1 à 9, qui contient en outre une charge organique ou inorganique, en particules.

11. Mélange de monomères conforme à la revendication 10, qui ne contient qu'une charge où la taille maximale des particules est inférieure à 600 nm.

12. Mélange de monomères conforme à l'une des revendications 1 à 11, qui contient
a) de 12,0 à 75 % en poids, et de préférence de 19 à 56 % en poids, d'un mélange de monomères conforme à l'une des revendications 1 à 3,
b) de 20 à 85 % en poids, et de préférence de 40 à 80 % en poids, d'une ou plusieurs charge(s),
c) de 0,05 à 4 % en poids, et de préférence de 0,1 à 2,0 % en poids, d'un amorceur de polymérisation par voie radicalaire,
d) et en option, de 0,1 à 5,0 % en poids, et de préférence de 0,1 à 2,0 % en poids, d'un ou plusieurs adjuvant(s).

13. Mélange de monomères conforme à l'une des revendications 1 à 12, pour utilisation intrabuccale en tant que ciment dentaire, composite d'obturation, matériau de revêtement ou matériau de placage (facette).

14. Utilisation d'un mélange de monomères conforme à l'une des revendications 1 à 12 pour la fabrication d'incrustations « in-lay » ou « on-lay », de couronnes ou de bridges.
